# EUROPEAN PATENT APPLICATION

(11) **EP 1 433 448 A1**
(43) Date of publication of application: **30.06.2004**
(21) Application number: 02717103.2
(22) Date of filing: 10.04.2002
(51) Int. Cl.: A61F 7/00, A61F 7/12, A61M 25/00

(54) **HEATING BALLOON−TIP CATHERTER AND ITS HEATING METHOD**

(30) Priority: 05.10.2001 JP 2001310450
(71) Applicant: Nihon Medix Co., Ltd., Matsudo-shi, Chiba 271-0065 (JP)
(72) Inventor: HASEBE, Kazunari c/o Nihon Medix Co., Ltd., Matsudo-shi, Chiba 271-0065 (JP)
(74) Representative: Thoma, Michael
(86) International application number: PCT/JP2002/003584
(87) International publication number: WO 2003/030789

(57) **Abstract**

A high-frequency heating electrode 3 and a temperature sensor 4 are disposed within a balloon 2 attached to a distal end portion of a catheter main body 1. With the balloon 2 being in abutment with a pulmonary vein opening 12a, a heating liquid within the balloon 2 is heated by the electrode 3, whereby the pulmonary vain opening 12a is annularly cauterized.

A reservoir 41 is connected to a connector 20 which is connected to a proximal end portion of the catheter main body 1. By reciprocally driving a diaphragm 42 of the reservoir 41 by a motor 43, a heating liquid within the reservoir 41 is vibrated, and the vibrations are transmitted to the heating liquid within the balloon 2 via a heating liquid within the connector 20 and a heating liquid within the catheter main body 1. While the heating liquid heated to a high temperature by the electrode 3 attempts to rise to gather at an upper portion within the balloon 2, since the heating liquid within the balloon 2 is stirred by the vibrations, the balloon 2 is heated uniformly.

## Description

### Technical Field

The present invention relates to a heating balloon-tip catheter apparatus and a heating method therefore.

### Background Art

In some of balloon-tip catheters in which a balloon is attached to a distal end portion of a catheter main body, a heating means is equipped within a balloon, and a heating liquid filled within the balloon is heated by the heating means so as to locally cauterize body tissues with which the balloon is brought into contact for treatment. Then, a thermocouple is also provided within the balloon as a temperature sensor for detecting the temperature of the heating liquid (for example, Japanese Unexamined Patent Publication (Kokai) No. 5-293183, and Japanese Unexamined Patent Publication (Kokai) No. 9-30638).

With the aforesaid heating balloon-tip catheter apparatus, there is a case where a considerably wide range of area of the balloon is desired to be used to cauterize the body tissues. To be specific, for example, the existence of an abnormal path (an accessory conduction path) of electric signal paths for moving the heart in a pulmonary vein causes symptoms such as vertigo and sick sensation which are referred to as atrial fibrillation, and the atrial fibrillation then causes the deterioration of cardiac incompetence or constitutes a serious cause for a brain infarction. In order to cut off the abnormal conduction path formed in the pulmonary vein, an edge portion of an opening of the pulmonary vein which is made to open toward the left ventricle (an opening of a pulmonary) is desirably cauterized totally, that is, annularly.

As has been described above, in a case where the heating liquid within the balloon is heated while attempting to maintain the temperature detected by.the temperature sensor equipped within the balloon to a predetermined temperature with the balloon being brought into an annular contact with the opening of the pulmonary vein as a whole, it has been found that there occurs a case where the cauterized condition of the opening of the pulmonary vein in a circumferential direction differs considerably. Namely, it has been found that there occurs a case where, on one hand, some portions of the opening of the pulmonary vein are heated excessively, and on the other hand, other portions of the pulmonary vein opening are heated insufficiently.

As the result of an investigation for a cause for the difference in heated condition by the difference in location where the balloon is brought into contact, it has been found that the difference in heated condition is caused by the fact that the heating liquid within the balloon is not heated uniformly, and hence, the temperature at an upper portion within the balloon becomes high, whereas the temperature at a lower position becomes low. Namely, it has been found that since the heating liquid heated by the heating means attempts to gather at a higher position within the balloon as the temperature thereof rises, the temperature at an upper portion of the balloon becomes considerably higher than the temperature of a lower portion of the balloon.

As has been described above, that the temperature of the balloon is not uniform results in an assumption that even if the balloon is brought into a local contact with the body tissues for cauterization, the temperature of the local contact portion does not reach the predetermined temperature, and a certain countermeasure is desired to be taken in this respect.

The present invention was made in these situations, and an object thereof is to provide a heating balloon-tip catheter apparatus which is adapted to heat the balloon uniformly in every part thereof and a heating method therefore.

### Disclosure of the Invention

With a view to attaining the object, according to an apparatus of the invention, the following approach is adopted for resolution. Namely, as is described in a first aspect of the invention, it is possible to provide a heating balloon-tip catheter apparatus in which a balloon is attached to a distal end portion of a catheter main body and heating means is equipped within the balloon, the balloon-tip catheter apparatus being characterized by provision of a vibrations imparting means connected to a proximal end portion of the catheter main body and adapted to vibrate a heating liquid filled within the balloon. Thus, the heating liquid within the balloon is stirred by the vibrations, whereby the temperature in the balloon can be made uniform everywhere.

On the premise of the aforesaid approach for resolution, the following approaches for resolution can be adopted in conjunction therewith.

It is possible that the vibrations imparting means is adapted to impart vibrations to the heating liquid within the balloon via a heating liquid filled within the catheter main body. In this case, vibrations can easily be imparted to the heating liquid within the balloon via the heating liquid within the catheter main body.

It is possible that the vibrations imparting means comprises;
a variable-volume reservoir connected to the proximal end of the catheter main body and having the heating liquid filled in an interior thereof, and
a driving means for changing the volume of the variable-volume reservoir. In this case, the heating liquid within the balloon can be vibrated via the heating liquid within the catheter main body by vibrating the heating liquid within the reservoir. Above all, since a portion such as the reservoir to which vibrations are imparted is provided separately exclusively for that purpose, the impartation of vibrations is preferably ensured and facilitated.

It is possible that the reservoir is set such that the volume thereof is changed by reciprocating motions of a movable member, and that the driving means is set so as to reciprocally driving the movable member. In this case, vibrations can be imparted to the heating liquid within the balloon with the simple approach in which the movable member is reciprocated.

It is possible that the reservoir is formed into something like a resiliently deformable tube so that the volume of the reservoir is changed by resilient deformation of the tube, and that the driving means is set to reciprocate so as to enable the resilient deformation of the reservoir repeatedly. In this case, since vibrations are imparted by making use of the resilient deformation of the resilient deformable tube, the construction of the vibrations imparting portion is made simple, and since the reservoir is formed into the tube, when considering a disposal, it is preferable in reducing the costs.

It is possible that a connector having a plurality of branch passageways is connected to the proximal end portion of the catheter main body, and that the vibrations imparting means is connected to a predetermined branch passageway of the plurality of branch passageways of the connector which is selectively made to communicate with or cut off from an interior of the catheter main body by an open-close valve. In this case, the vibrations imparting means can be connected to the proximal end portion of the catheter main body by making effective use of the connector adapted to be connected to the catheter main body. In addition, since the vibrations imparting means is connected to the branch passageway having the open-close valve, it is ensured that the vibrations imparting means is cut off from the interior of the catheter main body by closing the open-close valve except when vibrations need to be imparted.

It is possible that the predetermined branch passageway to which the vibrations imparting means is connected is made to be a branch passageway for supplying at least a contrast medium into the catheter main body. In this case, the vibrations imparting means can be connected by making effective use of the branch passageway through which the contrast medium is supplied.

It is possible that the heating means is made to be a high-frequency heating electrode, and that a thermocouple as a temperature sensor is equipped within the balloon. In this case, a common high-frequency heating electrode can be used as the heating means, and a common thermocouple can be used as the temperature sensor.

With a view to attaining the aforesaid object, according to the invention, the following approaches for resolution are adopted. Namely, as is described in a ninth aspect of the invention, it is possible to provide a heating method for a heating balloon-tip catheter apparatus in which a balloon is attached to a distal end portion of a catheter main body and a heating means is equipped within the balloon, the heating method being characterized in that with interiors of the balloon and the catheter main body being substantially filled with a heating liquid, vibrations are imparted to the heating liquid in the interior of the catheter main body from a proximal end portion side of the catheter main body (which correspond to the second aspect of the invention).

On the premise of the aforesaid approach for resolution, the following approach for resolution can be adopted in conjunction therewith.

It is possible that a reservoir filled with a heating liquid is connected to a proximal end portion of the catheter main body, and that vibrations are imparted to the heating liquid within the reservoir (this corresponding to the third aspect of the invention).

### Brief Description of the Drawings

Fig. 1 is an overall system diagram depicting one embodiment of the invention,
Fig. 2 is a main portion side cross-sectional view depicting a condition in which an opening of a pulmonary vein is cauterized,
Fig. 3 is a cross-sectional view taken along the line X3-X3 in Fig. 2,
Fig. 4 is a main portion side sectional view depicting another embodiment of the invention, and
Fig. 5 is a main portion side sectional view depicting a further embodiment of the invention.

### Best Mode for Carrying out the Invention

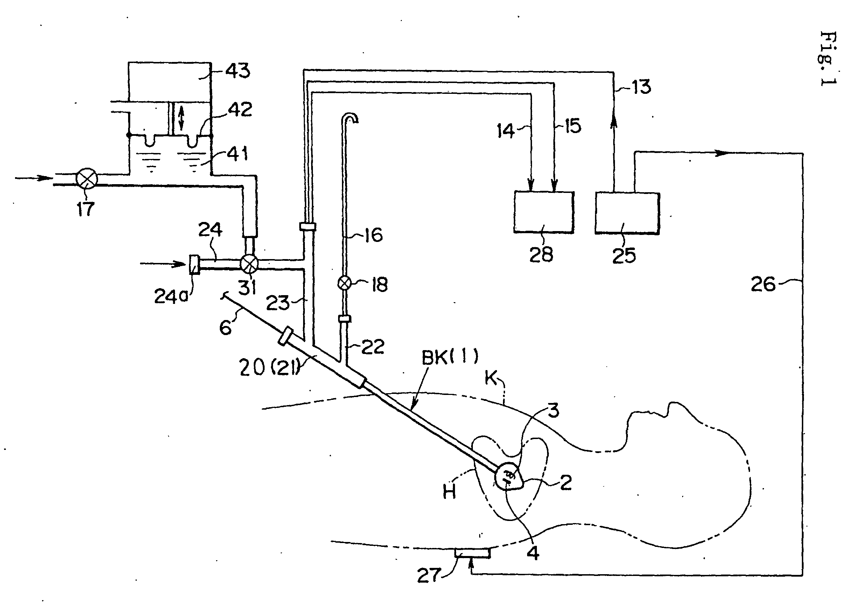

In Fig. 1, a catheter BK is intravenously inserted to a predetermined location of the heart H of a patient K from the outside of the body. The catheter is a heating balloon-tip catheter, and a balloon 2 is attached to a distal end portion of a tube-like catheter main body 1. As will be described later on, a high-frequency heating electrode 3 as a heating means and a thermocouple 4 as a temperature sensor are provided in this balloon 2.

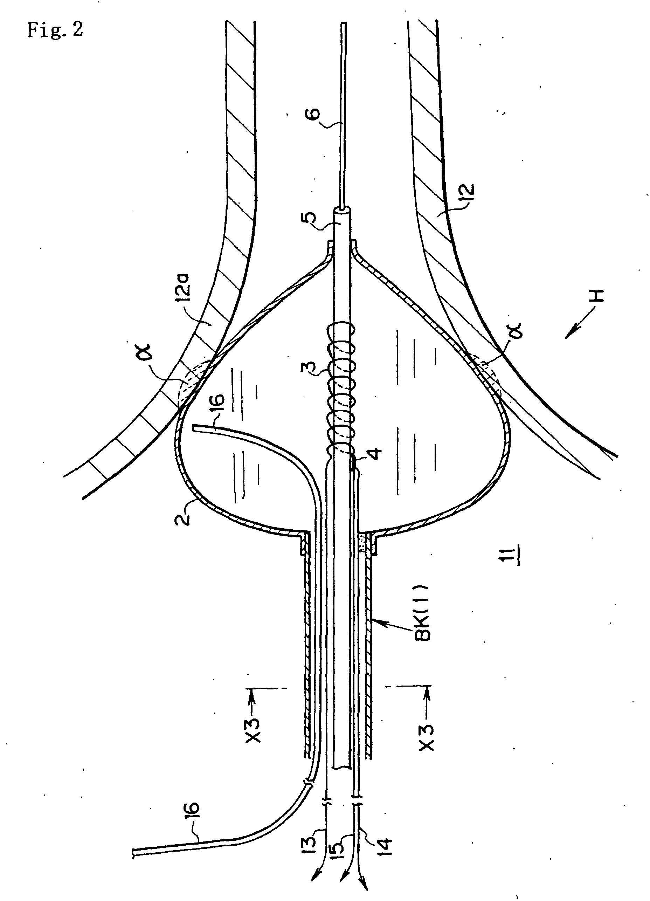

In Fig. 2, a guide tube 5 which is sufficiently thinner than the catheter main body 1 is equipped within the catheter main body 1. This guide tube 5 is made to be substantially as long as the catheter main body 1, and a distal end portion of the guide tube 5 is caused to slightly protrude from a distal end portion of the catheter main body 1. A guide wire 6 is allowed to pass through the guide tube 5.

The aforesaid balloon 2 is attached in such a manner as to extend between the distal end portion of the catheter main body 1 and the distal end portion of the guide tube 5. In the balloon 2, the heating electrode 3 is disposed in such a manner as to wind around the guide tube 5, and the thermocouple 4 is fixed to the guide tube 5.

Fig. 2 shows a condition in which an accessory conduction path from a pulmonary vein 12 which constitutes a cause for atrial fibrillation is cauterized by the heating balloon-tip catheter. Namely, Fig. 2 shows a condition in which the distal end portion of the catheter main body 1, that is, the balloon 2 is positioned in the left ventricle 11, and the balloon 2, which is inflated, is brought into annular contact with a pulmonary vein opening 12a which constitutes an opening edge. portion of the pulmonary vein towards the left ventricle 11. In Fig. 2, a portion to be cauterized is an annular portion resulting when the balloon 2 is brought into annular contact with the pulmonary vein opening 12a, and this portion to be cauterized is denoted by reference character α.

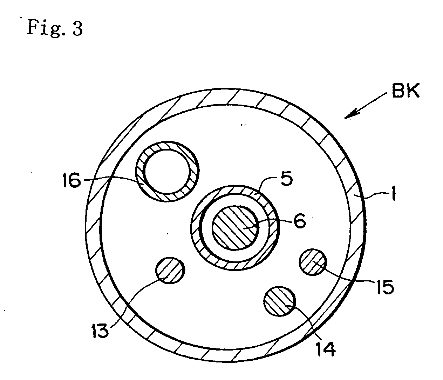

A wiring 13 extending from the heating electrode 3 and a pair of wirings 14, 15 which extend from the thermocouple 4 are such as to pass through the catheter main body 1 to extend finally to the outside of the body, as will be described later on. Furthermore, an air vent tube 16 for discharging air inside the balloon 2 is disposed within the catheter main body 1. This air vent tube 16 is made to open at a distal end portion thereof to the interior of the balloon 2 at a higher position within the balloon 2, and the other end portion thereof is made to open to the atmosphere at a higher position outside the body. Note that a layout condition of the respective constituent elements 5, 6, 13 to 16 is schematically shown in Fig. 3.

Referring again to Fig. 1, a connector 20 is connected to a proximal end portion of the catheter main body 1, that is, a portion which is located outside the body. This connector 20 has a main body passageway 21 which extends substantially coaxially with the catheter main body 1, a first branch passageway 22 and a second branch passageway 23 which branch from the main body passageway 21, respectively, and a third branch passageway 24 which branches further from the second branch passageway 23.

The guide wire 6 passes through the main body passageway 21 of the connector 20 and is then allowed to extend to the outside of the body. Note that a blood stop valve for preventing the reverse flow of blood is provided at a location where the guide wire 6 is guided to the outside of the body.

The respective wirings 13, 14, 15 are guided to the outside of the body from the second branch passageway 23, and this location where the wirings are guided to the outside of the body is sealed. The wiring 13 of the heating electrode 3 is connected to a high-frequency (a high-frequency voltage) generator 25. A wiring 26 which constitutes a pair to the wiring 13 extends from the high-frequency generator 25, and this wiring 26 is connected to an extracorporeal electrode 27 which is brought into contact with the surface of the body. With the electrode 3 within the balloon 2 being positioned at a predetermined location within the body 'as shown in Fig. 2 and the extracorporeal electrode 27 being brought into contact with the surface of the body, the high-frequency generator 25 is activated, whereby a high-frequency energization is implemented between the two electrodes 3 and 27 to thereby heat the heating electrode 3.

The wirings 14, 15 which extend from the thermocouple 4 are both connected to a temperature measuring device 28 which employs a voltmeter. A difference in voltage according to a temperature within the balloon 2 is inputted into the temperature measuring device 28 via the two wirings 14, 15, whereby a temperature within the balloon 2 is detected and indicated.

A connecting portion 24a is constructed at an end portion of the third branch passageway 24 so that a syringe for supplying a contrast medium or, for example, a physiological saline as a heating liquid is connected thereto. In addition, while an open-close valve is provided at the connecting portion 24a which is adapted to be pressed to open when a syringe is connected to the connecting portion 24a and to automatically close when the connection is released, an open-close valve which is manually opened and closed may be provided instead.

A reservoir 41 is connected to an intermediate position along the length of the third branch passageway 24 via a selector valve 31. The selector valve 31 is such as to selectively connect the catheter main body 1 to the connecting portion 24a or the reservoir 41 to thereby function as an open-close valve which selectively establishes a communication between the reservoir 41 and the catheter main body 1 or cuts off such a communication. The reservoir 41 stores, for example, a physiological saline as a heating liquid, and a top wall is constituted by a diaphragm 42 functioning as a movable member. Vibrations are imparted to the heating liquid stored in the reservoir 41 by vertically reciprocating the diaphragm 42 by a reciprocating motor 43 as a driving means. Thus, in this embodiment, the reservoir 41 having the diaphragm 42 and the motor 43 constitute a vibrations imparting means.

Next, the function of the heating balloon-tip catheter that is constructed as has been described heretofore will be described. Firstly, the guide wire 6 is inserted intravenously from the outside to the inside of the body, and the distal end portion thereof passes through the left ventricle 11 to thereby be positioned at a location where the guide wire 6 is slightly inserted into the pulmonary vein 12. The catheter main body 1 is inserted into the body along the guide wire 6 which functions as a guide (in such a manner that the guide wire 6 is passed through the interior of the guide tube 5) (with the balloon 2 being kept contracted).

When the arrival of the balloon 2 at the left ventricle 11 and moreover the location thereof near the pulmonary vein opening 12a are verified from the outside of the body by making use of a contrast medium which is supplied from the connecting portion 24a to the balloon 2, air is send under pressure from, for example, the connecting portion 24a so as to inflate the balloon 2, whereby the condition shown in Fig. 2 results where the balloon 2 is brought into strong contact with the pulmonary vein opening 12a. Note that in inflating the balloon 2, an open-close valve 18 connecting to the air vent tube 16 can be closed.

From the condition shown in Fig. 2, a heating liquid is supplied into the balloon 2 from, for example, the connecting portion 24a so as to fill interiors of the balloon 2 and the catheter main body 1 and furthermore the connector 21 with the heating liquid so supplied. In supplying the heating liquid into the balloon 2, air within the balloon 2 is effectively discharged to the outside through the air vent tube 16 (with the open-close valve 18 being opened), so that the supply of the heating liquid into the balloon 2 is implemented smoothly. In addition, when air remains within the balloon 2, after air so remaining within the balloon 2 is drawn to the outside from, for example, the connecting portion 24a, the supply of the heating liquid is resumed, and the operation like this is repeated as required.

The heating liquid can be supplied to the balloon 2 via the reservoir 41. Namely, as shown in Fig. 1, a supply source of heating liquid (not shown) is connected to the reservoir 41 via an open-close valve 17, and the selector valve 31 and the open-close valve 17 are opened, so that the heating liquid from the heating liquid supply source may be supplied to the balloon 2 via the reservoir 41.

When it is verified that the interiors of the balloon 2, the catheter main body 1 and the connector 21 are filled with the heating liquid, the high-frequency generator 25 is activated, heating by the heating electrode 3 is started. The operating condition of the high-frequency generator 25 is feedback controlled so that the temperature in the balloon 2 becomes a predetermined temperature (for example, 60°C) or the temperature detected by the thermocouple 4 becomes the predetermined temperature.

When heating by the heating electrode 3 is carried out, the selector valve 31 is directed to the reservoir 41 side, and the motor 43 is activated. When the motor 43 is activated, the diaphragm 42 is reciprocated, the heating liquid within the reservoir 41 is vibrated (for example, at several tens HZ). The vibration of the heating liquid within the reservoir 41 is transmitted to the heating liquid within the balloon 2 via the heating liquid within the connector 21 and the heating liquid within the catheter main body 1, whereby the heating liquid within the balloon 2 is stirred. Thus, while the heating liquid heated to a high temperature by the heating electrode 3 attempts to gather at an upper portion within the balloon 2, due to the heating liquid being stirred by vibrations, a uniform temperature results in every part of the interior of the balloon 2.

The pulmonary vein opening 12a is cauterized by the heated balloon 2. Since the uniform temperature results in every part of the interior of the balloon 2, the pulmonary vein opening 12a is cauterized uniformly all along the full circumferential length thereof.

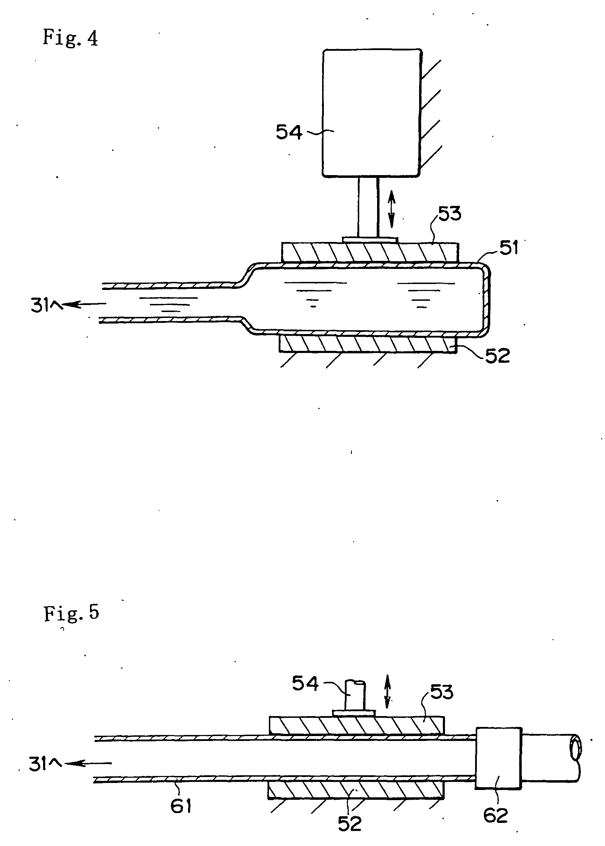

Fig. 4 shows another embodiment of the invention. In this embodiment, a reservoir 51, which corresponds to the reservoir 41, is such as to use a resiliently deformable tube (for example, a silicone tube). Namely, the reservoir 51 connected to a selector valve 31 is made to be a closed container by a closed elongate tube. Then, the reservoir 51 is placed on a fixed member 52. A pressing member 53 is positioned above the reservoir 51, so that the tube-like reservoir 51 can be strongly compressed from radial directions by the pressing member 53 and the fixed member 52. The pressing member 53 is reciprocally driven in vertical directions by a reciprocating motor 54, whereby when the motor 54 is activated so as to reciprocally drive the pressing member 53 in the vertical directions, the tube-like reservoir 51 repeats alternately a condition where the tube-like reservoir 51 is strongly compressed in the radial directions and a condition where the tube-like reservoir 51 is released from the compressed condition, whereby a heating liquid within the tube-like reservoir 51 is vibrated.

Fig. 5 is a modification to the embodiment shown in Fig. 4, in which an end portion of a tube-like reservoir 61 which is opposite to an end thereof where a selector valve 31 is connected is connected to a supply source of heating liquid (not shown) via an open-close valve 62. In this case, with the open-close valve 62 being closed, a motor 54 is activated, whereby, as in the case shown in Fig. 4, a heating liquid within the tube-like reservoir 61 is vibrated. In the case shown in Fig. 5, when supplying a heating liquid into a balloon 2, the open-close valve 62 and the selector valve 31 are both opened, so that the heating liquid can be supplied from the supply source of heating liquid into the balloon 2 via the reservoir 61.

While the embodiments of the invention have been described heretofore, the invention is not limited thereto but may be modified variously without departing from the scope of claims of the invention. For example, there can be provided a construction in which the heating electrode 3 can be made to function as one of the conductors of the thermocouple 4 as well, so that one of the wirings 14, 15 can be abolished (for example, in the Japanese unexamined Patent Publication No. 5-293183, a construction is disclosed in which a wiring of a heating electrode is also used as one of wirings of a thermocouple). It is possible to provide no separate air vent tube 16 (in particular, in order to reduce an outside diameter of the catheter main body 1). Of course, the object of the invention includes not only that clearly expressed but also those implicitly suggested to provide what is represented as being substantially preferable or as advantages. Furthermore, the invention can be represented as a heating method.

According to the invention, the balloon can be uniformly heated in every part therein.

## Claims

1. A heating balloon-tip catheter apparatus in which a balloon is attached to a distal end portion of a catheter main body and heating means is equipped within the balloon, the balloon-tip catheter apparatus being **characterized by** provision of vibrations imparting means connected to a proximal end portion of the catheter main body and adapted to vibrate a heating liquid filled within the balloon.

2. A heating balloon-tip catheter apparatus as set forth in Claim 1, wherein
the vibrations imparting means is adapted to impart vibrations to the heating liquid within the balloon via a heating liquid filled within the catheter main body.

3. A heating balloon-tip catheter apparatus as set forth in Claim 2, wherein the vibrations imparting means comprises,
a variable-volume reservoir connected to the proximal end of the catheter main body and having the heating liquid filled in an interior thereof, and
driving means for changing the volume of the variable-volume reservoir.

4. A heating balloon-tip catheter apparatus as set forth in Claim 3, wherein
the reservoir is set such that the volume thereof is changed by reciprocating motions of a movable member, and wherein
the driving means is set so as to reciprocally driving the movable member.

5. A heating balloon-tip catheter apparatus as set forth in Claim 3, wherein
the reservoir is formed into something like a resiliently deformable tube so that the volume of the reservoir is changed by resilient deformation of the resilient deformable tube, and wherein
the driving means is set to reciprocate so as to enable the resilient deformation of the reservoir repeatedly.

6. A heating balloon-tip catheter apparatus as set forth in any of Claims 1 to 6, wherein
a connector having a plurality of branch passageways is connected to the proximal end portion of the catheter main body, and wherein
the vibrations imparting means is connected to a predetermined branch passageway of the plurality of branch passageways of the connector which is selectively made to communicate with or cut off from an interior of the catheter main body by an open-close valve.

7. A heating balloon-tip catheter apparatus as set forth in Claim 6, wherein
the predetermined branch passageway to which the vibrations imparting means is connected is made to be a branch passageway for supplying at least a contrast medium into the catheter main body.

8. A heating balloon-tip catheter apparatus as set forth in any of Claims 1 to 7, wherein
the heating means is made to be a high-frequency heating electrode, and wherein
a thermocouple as a temperature sensor is equipped within the balloon.

9. A heating method for a heating balloon-tip catheter apparatus in which a balloon is attached to a distal end portion of a catheter main body and heating means is equipped within the balloon, the heating method being **characterized in that**;
with interiors of the balloon and the catheter main body being substantially filled with a heating liquid, vibrations are imparted to the heating liquid in the interior of the catheter main body from a proximal end portion side of the catheter main body.

10. A heating method for a heating balloon-tip catheter apparatus as set forth in Claim 9, wherein
a reservoir filled with a heating liquid is connected to a proximal end portion of the catheter main body, and wherein
vibrations are imparted to the heating liquid within the reservoir.
